# EUROPEAN PATENT APPLICATION

(11) **EP 4 789 552 A1**
(43) Date of publication of application: **12.08.2026**
(21) Application number: 24874733.9
(22) Date of filing: 04.10.2024
(51) Int. Cl.: A23L 33/125, A23L 33/17, A23L 33/175, A23L 33/185, A23L 33/19

(54) **FOOD FOR IMPROVING INTESTINAL ENVIRONMENT**

(30) Priority: 05.10.2023 JP 2023173408
(71) Applicant: AJINOMOTO CO., INC., Chuo-ku Tokyo 104-8315 (JP)
(72) Inventor: KAMEYAMA, Keishi, Kawasaki-shi, Kanagawa 210-8681 (JP); FUJII, Takayoshi, Tokyo 104-8315 (JP)
(74) Representative: Strehl & Partner mbB
(86) International application number: PCT/JP2024/035580
(87) International publication number: WO 2025/075133

(57) **Abstract**

The present invention aims to provide a food that can increase the amount of indole-3-propionic acid produced by intestinal bacteria.

A food for improving the intestinal environment, containing a combination of (1) an indigestible polysaccharide or a composition containing an indigestible polysaccharide and (2) an indigestible protein or a composition containing an indigestible protein. A food for improving the intestinal environment, containing at least one selected from the group consisting of an enteric composition containing tryptophan or a salt thereof, an indigestible protein containing tryptophan as a constituent amino acid, and a composition containing an indigestible protein containing tryptophan as a constituent amino acid.

## Description

### [Technical Field]

The present invention relates to a food that can increase the amount of indole-3-propionic acid, produced by enteric bacteria, by improving the intestinal environment.

### [Background Art]

Advances in studies of intestinal bacteria in recent years have revealed that substances produced by the metabolic conversion of dietary components by intestinal bacteria play an important role in the health status and onset of disease in the host.

Indole-3-propionic acid (hereinafter referred to as "IPA") is produced in the intestine when tryptophan derived from the diet ingested by the host is metabolized and converted by intestinal bacteria such as Clostridium sporogenes and the like. IPA is known to have beneficial effects on the health of host (for example, anti-obesity action (Non Patent Literature 1), the intestinal barrier function-protective action (Non Patent Literature 2), antioxidant action (Non Patent Literature 3), protective effects against nerve damage (Non Patent Literature 4), endoplasmic reticulum stress improving effect (Non Patent Literature 5), muscle mass increase action (Non Patent Literature 6), bone formation promoting action (Non Patent Literature 7), antibacterial action (Non Patent Literature 8), and glucose tolerance improving effect (Non Patent Literature 9)). Furthermore, regarding intestinal permeability, which is considered one of the underlying causes of obesity and type 2 diabetes, it has been revealed that administration of Clostridium sporogenes bacteria and IPA to mice activates and improves the nuclear receptor PXR in intestinal epithelial cells (Non Patent Literature 10). This suggests that the effect of IPA on improving intestinal permeability suppresses obesity and impaired glucose tolerance.

Therefore, there is a need for the development of foods that can increase IPA, which is expected to have the above-mentioned beneficial effects.

However, since IPA itself is not approved as a food additive, it cannot be used directly in food. Also, even if tryptophan, which is a precursor of IPA, is ingested orally, it is rapidly absorbed, making it difficult to reach the large intestine where many intestinal bacteria reside.

Indigestible starch is known to increase the amount of IPA produced in the intestine (Non Patent Literature 11), and further food development is demanded.

### [Citation List]

### [Non Patent Literature]

[Non Patent Literature 1]
   Nutrients 2019, 11, 591
[Non Patent Literature 2]
   Exp Mol Med.2019 Sep 10; 51(9):1-14
[Non Patent Literature 3]
   Membranes 2021, 11, 571.
[Non Patent Literature 4]
   Nature 607, 585-592 (2022)
[Non Patent Literature 5]
   Biochem Biophys Res Commun. 2019, 517(4), 623-628
[Non Patent Literature 6]
   Int. J. Mol. Sci. 2021, 22, 12435
[Non Patent Literature 7]
   Theranostics 2021, Vol. 11, Issue 17
[Non Patent Literature 8]
   Life Sci. 2021, 285:120003
[Non Patent Literature 9]
   FEBS J. 2022 Oct; 289(19):5985-6004
[Non Patent Literature 10]
   Immunity, 2014; 41(2): 293-310
[Non Patent Literature 11]
   FASEB J. 2019 Jul; 33(7):8033-8042

### [Summary of Invention]

### [Technical Problem]

The present invention aims to provide a food that can increase the amount of IPA produced by intestinal bacteria.

### [Solution to Problem]

The present inventors have conducted intensive studies in an attempt to solve the aforementioned problems and found that ingestion of tryptophan, which is a precursor of IPA, as an enteric composition can increase the amount of IPA produced by intestinal bacteria, that phenylalanine is essential for promoting the growth of Clostridium sporogenes, which is an intestinal bacterium that produces IPA, that ingestion of an enteric composition containing tryptophan and phenylalanine in combination can increase the amount of IPA produced by intestinal bacteria further remarkably than ingestion of an enteric composition of tryptophan alone, and that ingestion of an enteric composition of tryptophan in combination with indigestible polysaccharides such as oligosaccharide and the like can increase the amount of IPA produced by intestinal bacteria further remarkably than ingestion of an enteric composition of tryptophan alone.

Furthermore, the present inventors have found that ingestion of indigestible proteins such as soybean protein and the like in combination with indigestible polysaccharides such as oligosaccharide and the like can remarkably increase the amount of IPA produced by intestinal bacteria.

The present inventors have conducted further studies based on these findings and completed the present invention.

Accordingly, the present invention provides the following.
[1] A food for improving the intestinal environment, comprising a combination of (1) an indigestible polysaccharide or a composition containing an indigestible polysaccharide and (2) an indigestible protein or a composition containing an indigestible protein (also referred to as the first invention in the present specification).
[2] The food of the above-mentioned [1], further comprising (3) an enteric composition containing an amino acid or a salt thereof in combination.
[3] The food of the above-mentioned [1], wherein the indigestible polysaccharide or the composition containing an indigestible polysaccharide is oligosaccharide or a composition containing oligosaccharide.
[4] The food of the above-mentioned [1], wherein the indigestible protein or the composition containing an indigestible protein is a plant protein or a composition containing a plant protein.
[5] The food of the above-mentioned [1], wherein the indigestible protein or the composition containing an indigestible protein is selected from the group consisting of soybean protein, sake lees, freeze-dried tofu, buckwheat, wheat, rice, peas, and corn.
[6] The food of the above-mentioned [2], wherein the amino acid is tryptophan, or a combination of tryptophan and phenylalanine.
[7] The food of any one of the above-mentioned [1] to [6], for increasing the production of indole-3-propionic acid by intestinal bacteria in the intestine.
[8] A food for improving the intestinal environment, comprising at least one selected from the group consisting of an enteric composition containing tryptophan or a salt thereof, an indigestible protein containing tryptophan as a constituent amino acid, and a composition containing an indigestible protein containing tryptophan as a constituent amino acid (also referred to as the second invention in the present specification).
[9] The food of the above-mentioned [8], further comprising an enteric composition containing phenylalanine or a salt thereof in combination.
[10] The food of the above-mentioned [8], wherein the indigestible protein containing tryptophan as a constituent amino acid, or the composition containing an indigestible protein containing tryptophan as a constituent amino acid is selected from the group consisting of soybean protein, sake lees, freeze-dried tofu, buckwheat, wheat, rice, peas, and corn.
[11] The food of the above-mentioned [8], further comprising an indigestible polysaccharide or a composition containing an indigestible polysaccharide in combination.
[12] The food of any one of the above-mentioned [8] to [11], for increasing the production of indole-3-propionic acid by intestinal bacteria in the intestine.
[13] A method for improving the intestinal environment in a subject, comprising administering an effective amount of a food comprising a combination of (1) an indigestible polysaccharide or a composition containing an indigestible polysaccharides, and (2) an indigestible protein or a composition containing an indigestible protein to the subject.
[14] The method of the above-mentioned [13], wherein the aforementioned food is further combined with (3) an enteric composition containing an amino acid or a salt thereof.
[15] The food of the above-mentioned [13], wherein the indigestible polysaccharide or the composition containing an indigestible polysaccharide is oligosaccharide or a composition containing oligosaccharide.
[16] The method of the above-mentioned [13], wherein the indigestible protein or the composition containing an indigestible protein is a plant protein or a composition containing a plant protein.
[17] The method of the above-mentioned [13], wherein the indigestible protein or the composition containing an indigestible protein is selected from the group consisting of soybean protein, sake lees, freeze-dried tofu, buckwheat, wheat, rice, peas, and corn.
[18] The method of the above-mentioned [14], wherein the amino acid is tryptophan, or a combination of tryptophan and phenylalanine.
[19] The method of any one of the above-mentioned [13] to [18], for increasing the production of indole-3-propionic acid by intestinal bacteria in the intestine.
[20] A method for improving the intestinal environment in a subject, comprising administering an effective amount of a food containing at least one selected from the group consisting of an enteric composition containing tryptophan or a salt thereof, an indigestible protein containing tryptophan as a constituent amino acid, and a composition containing an indigestible protein containing tryptophan as a constituent amino acid.
[21] The method of the above-mentioned [20], wherein the aforementioned food is further combined with an enteric composition containing phenylalanine or a salt thereof.
[22] The method of the above-mentioned [20], wherein the indigestible protein containing tryptophan as a constituent amino acid, or the composition containing an indigestible protein containing tryptophan as a constituent amino acid, is selected from the group consisting of soybean protein, sake lees, freeze-dried tofu, buckwheat, wheat, rice, peas, and corn.
[23] The method of the above-mentioned [20], wherein the aforementioned food is further combined with an indigestible polysaccharide or a composition containing an indigestible polysaccharide.
[24] The method of any one of the above-mentioned [20] to [23], for increasing the production of indole-3-propionic acid by intestinal bacteria in the intestine.
[25] Use of a food comprising a combination of (1) an indigestible polysaccharide or a composition containing an indigestible polysaccharide, and (2) an indigestible protein or a composition containing an indigestible protein, for improving the intestinal environment.
[26] The use of the above-mentioned [25], wherein the aforementioned food is further combined with (3) an enteric composition containing an amino acid or a salt thereof.
[27] The food of the above-mentioned [25], wherein the indigestible polysaccharide or the composition containing an indigestible polysaccharide is oligosaccharide or a composition containing oligosaccharide.
[28] The use of the above-mentioned [25], wherein the indigestible protein or the composition containing an indigestible protein is a plant protein or a composition containing a plant protein.
[29] The use of the above-mentioned [25], wherein the indigestible protein or the composition containing an indigestible protein is selected from the group consisting of soybean protein, sake lees, freeze-dried tofu, buckwheat, wheat, rice, peas, and corn.
[30] The use of the above-mentioned [26], wherein the amino acid is tryptophan, or a combination of tryptophan and phenylalanine.
[31] The use of any one of the above-mentioned [25] to [30], for increasing the production of indole-3-propionic acid by intestinal bacteria in the intestine.
[32] Use of a food comprising at least one selected from the group consisting of an enteric composition containing tryptophan or a salt thereof, an indigestible protein containing tryptophan as a constituent amino acid, and a composition containing an indigestible protein containing tryptophan as a constituent amino acid, for improving the intestinal environment.
[33] The use of the above-mentioned [32], wherein the aforementioned food is further combined with an enteric composition containing phenylalanine or a salt thereof.
[34] The use of the above-mentioned [32], wherein the indigestible protein containing tryptophan as a constituent amino acid, or a composition containing an indigestible protein containing tryptophan as a constituent amino acid, is selected from the group consisting of soybean protein, sake lees, freeze-dried tofu, buckwheat, wheat, rice, peas, and corn.
[35] The use of the above-mentioned [32], wherein the aforementioned food is further combined with an indigestible polysaccharide or a composition containing an indigestible polysaccharide.
[36] The use of any one of the above-mentioned [32] to [35], for increasing the production of indole-3-propionic acid by intestinal bacteria in the intestine.

### [Advantageous Effects of Invention]

The food for improving the intestinal environment of the present invention (first invention) is expected to remarkably increase the amount of IPA produced by intestinal bacteria in the intestine of a person who has ingested the food, by combining (1) an indigestible polysaccharide or a composition containing an indigestible polysaccharide (for example, fructooligosaccharide or a composition containing a fructooligosaccharide), and (2) an indigestible protein or a composition containing an indigestible protein (for example, soybean protein or a composition containing soybean protein), compared to when (1) or (2) is ingested alone. As a result, it is expected to increase the blood IPA concentration (see Experimental Example 4).

In addition, the food for improving the intestinal environment of the present invention (first invention) can be expected to improve intestinal permeability by combining (1) an indigestible polysaccharide or a composition containing an indigestible polysaccharide (for example, fructooligosaccharide or a composition containing a fructooligosaccharide), and (2) an indigestible protein or a composition containing an indigestible protein (for example, soybean protein or a composition containing soybean protein) (see Experimental Example 5).

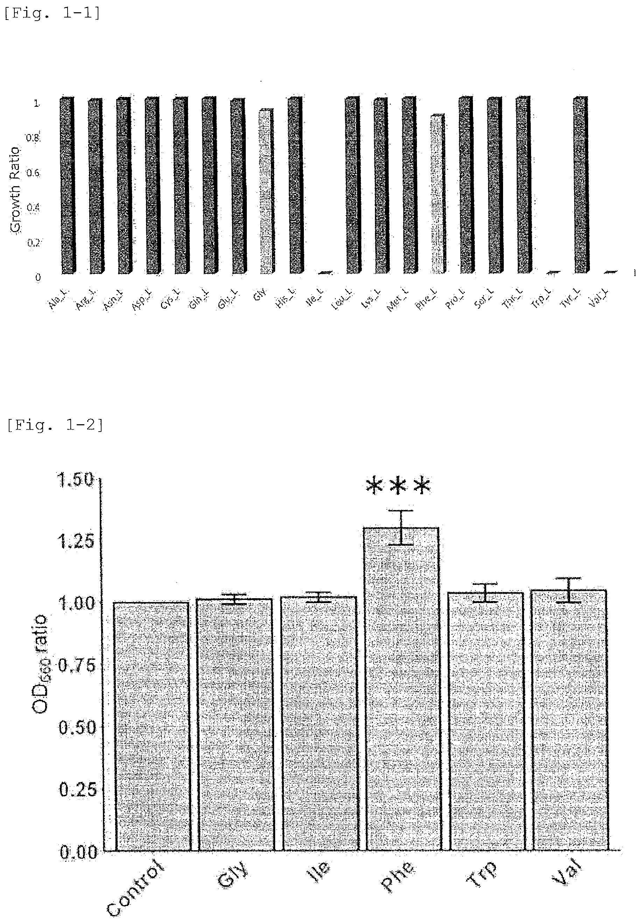

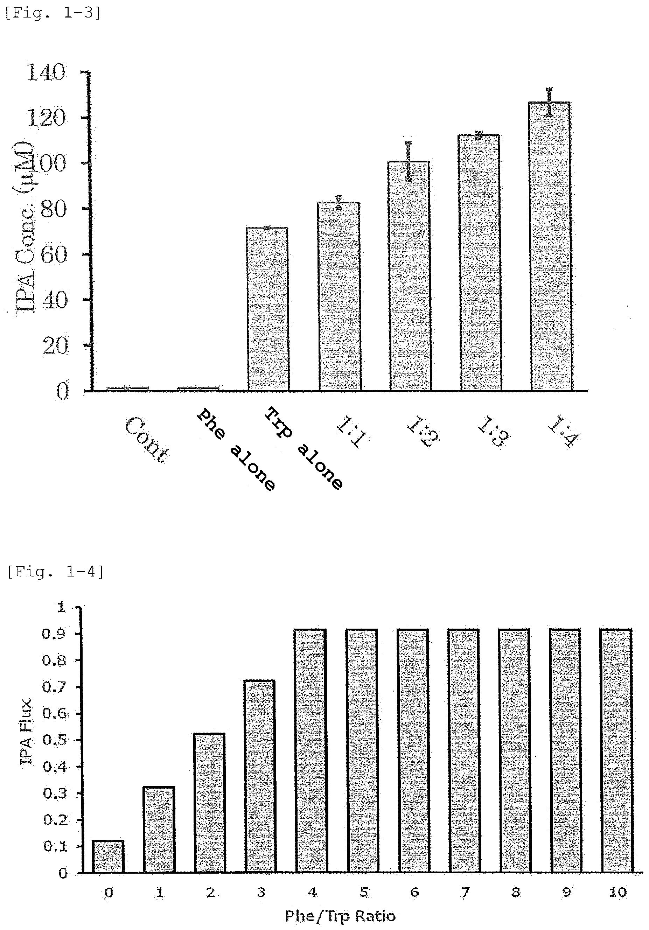

The food for improving the intestinal environment of the present invention (second invention) contains at least one selected from the group consisting of an enteric composition containing tryptophan or a salt thereof, an indigestible protein containing tryptophan as a constituent amino acid (e.g., soybean protein), and a composition containing an indigestible protein containing tryptophan as a constituent amino acid. It is expected that the food will remarkably increase the amount of IPA produced by intestinal bacteria in the intestine of a subject who ingested the food, thereby increasing the blood IPA concentration (see Experimental Example 1-2, Fig. 1-3).

In addition, the food for improving the intestinal environment of the present invention (second invention), when further combined with an enteric composition containing phenylalanine, is expected to further remarkably increase the production of IPA by intestinal bacteria, compared to the ingestion of only an enteric composition containing tryptophan, an indigestible protein containing tryptophan as a constituent amino acid (e.g., soybean protein), or a composition containing an indigestible protein containing tryptophan as a constituent amino acid. As a result, the food can be expected to increase blood IPA concentration (see Experimental Example 1-2, Figs. 1-3 and 1-4).

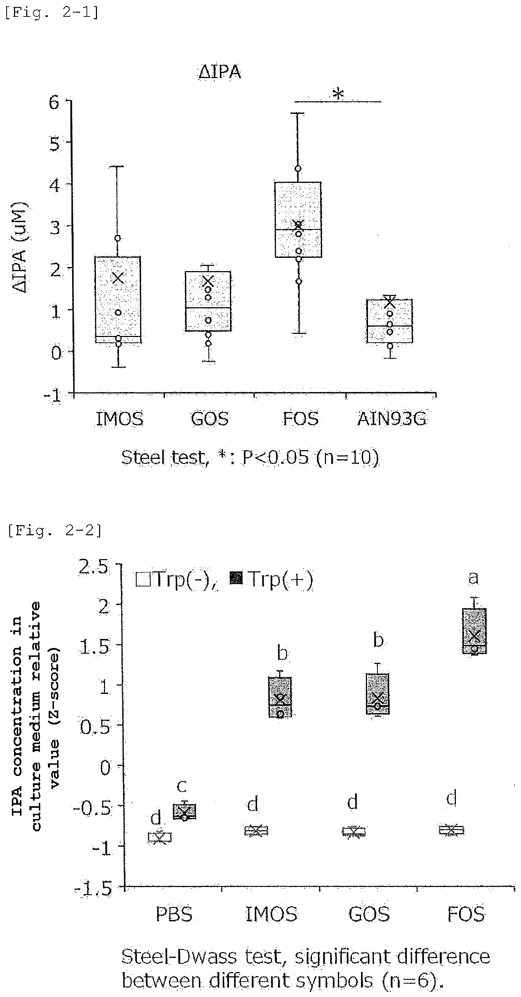

In addition, the food for improving the intestinal environment of the present invention (second invention), when further combined with an indigestible polysaccharide (e.g., fructooligosaccharide) or a composition containing an indigestible polysaccharide, can be expected to further remarkably increase the amount of IPA produced by intestinal bacteria, compared to the ingestion of only an enteric composition containing tryptophan, an indigestible protein containing tryptophan as a constituent amino acid (e.g., soybean protein), or a composition containing an indigestible protein containing tryptophan as a constituent amino acid. As a result, the food can be expected to increase blood IPA concentration (see Experimental Example 2-2, Fig. 2-2).

### [Brief Description of Drawings]

[Fig. 1-1]
   Fig. 1-1 is a graph showing the results of Experimental Example 1-1.
[Fig. 1-2]
   Fig. 1-2 is a graph showing the results of Experimental Example 1-1.
[Fig. 1-3]
   Fig. 1-3 is a graph showing the results of Experimental Example 1-2.
[Fig. 1-4]
   Fig. 1-4 is a graph showing the results of Experimental Example 1-2.
[Fig. 2-1]
   Fig. 2-1 is a graph showing the results of Experimental Example 2-1.
[Fig. 2-2]
   Fig. 2-2 is a graph showing the results of Experimental Example 2-2.
[Fig. 3]

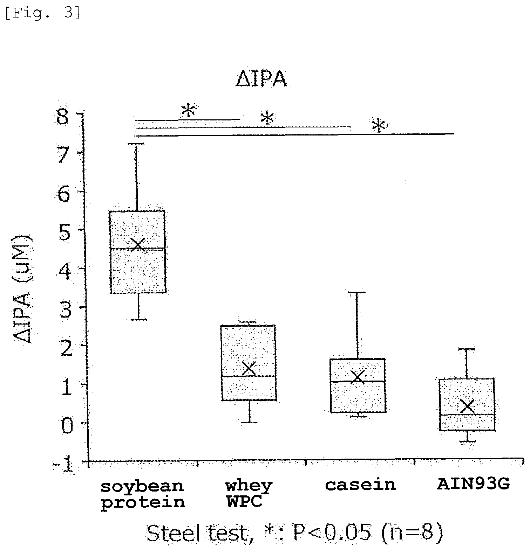

   Fig. 3 is a graph showing the results of Experimental Example 3.
[Fig. 4]

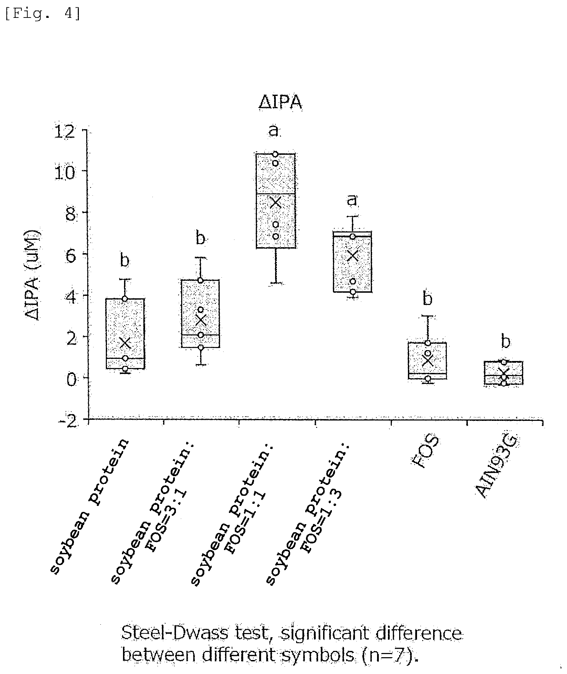

   Fig. 4 is a graph showing the results of Experimental Example 4.
[Fig. 5]

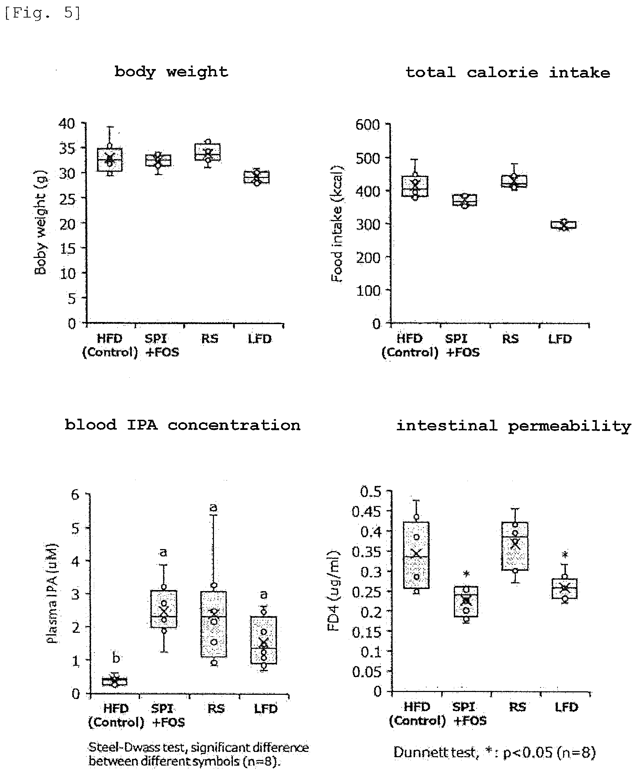

   Fig. 5 is a graph showing the results of Experimental Example 5.

### [Description of Embodiments]

### [First invention]

The food for improving intestinal environment of the present invention uses (1) an indigestible polysaccharide or a composition containing an indigestible polysaccharide, and (2) an indigestible protein or a composition containing an indigestible protein in combination.

The food for improving intestinal environment of the present invention is preferably further combined with (3) an enteric composition containing an amino acid or a salt thereof.

The food of the present invention is a combined use food of (1) and (2) (preferably a combined use food of (1), (2), and (3)) in which (1) and (2) are used in combination (preferably (1), (2), and (3) in combination).

In the food of the present invention, (1) and (2) (or (1), (2), and (3)) may be simultaneously formulated and contained in the same preparation (food), or (1) and (2) (or (1), (2), and (3)) may be formulated separately and ingested simultaneously or at different times by the same route or different routes. That is, the food of the present invention includes a food containing (1) and (2) (or (1), (2), and (3)) in one preparation, and a food combining (1) and (2) (or (1), (2), and (3)) formulated separately.

### (1) Indigestible polysaccharide or composition containing an indigestible polysaccharide

In the present specification, indigestible polysaccharide is a polysaccharide that is not easily digested or absorbed in the stomach or small intestine and reaches large intestine to be metabolized intestinal bacteria such as Clostridium sporogenes and the like.

In the present invention, the degree of polymerization of the indigestible polysaccharides is not less than 2 or not less than 3. In the present invention, the upper limit of the degree of polymerization of indigestible polysaccharides is not particularly limited and is, for example, not more than 100, preferably not more than 60, more preferably not more than 10, further preferably not more than 6, further more preferably not more than 5.

The degree of polymerization of the indigestible polysaccharides in the present invention is preferably 2 - 100, more preferably 2 - 60, further preferably 2 - 10, further more preferably 2 - 6.

Examples of the indigestible polysaccharides in the present invention include oligosaccharide, guar gum, indigestible maltodextrin, pectin, chitosan, alginic acid, inulin, and the like.

As (1) in the present invention, indigestible polysaccharides (e.g., oligosaccharide) can be used as they are or a composition containing an indigestible polysaccharides (e.g., oligosaccharide) can be used.

The oligosaccharide in the present specification has 2 to 10 monosaccharides bonded thereto.

Examples of the oligosaccharide in the present invention include coffee bean manno-oligosaccharide, lactosucrose oligosaccharide, galacto-oligosaccharide (GOS), fructo-oligosaccharide (FOS), soybean oligosaccharide, xylo-oligosaccharide and isomalto-oligosaccharide (IMOS), fructo-oligosaccharide, galacto-oligosaccharide, or isomalto-oligosaccharide is preferred, and fructo-oligosaccharide is particularly preferred. One or more kinds of oligosaccharides can be used in combination.

Oligosaccharide can be produced by a known method, and commercially available products can also be used.

Examples of the fructo-oligosaccharide include 1-kestose, nystose, 1-β-fructofuranosylnistose, and the like with polymerization degree of 3 - 5 in which 1 to 3 fructoses are bonded to sucrose. Specifically, for example, FOS: FUJIFILM Wako Pure Chemical Industries, Ltd., #064-02385 can be mentioned.

Examples of the galacto-oligosaccharide include galactosyl lactose with polymerization degree of 2 - 6, which has a structure in which one or more galactose molecules are linked to lactose via β-1,4 bonds. Specifically, for example, GOS: FUJIFILM Wako Pure Chemical Industries, Ltd., #076-05945 can be mentioned.

Examples of the isomalto-oligosaccharide include branched oligosaccharides having glucose as a constituent sugar and one or more of α-1,6, α-1,2, and α-1,3 bonds in a molecule, such as isomaltose, nigerose, kordibiose, panose, isomalttriose, isomalttetraose, and the like. Specifically, for example, IMOS: FUJIFILM Wako Pure Chemical Corporation, #090-03485 can be mentioned.

The form of the composition containing an indigestible polysaccharides (e.g., oligosaccharide) of the present invention is not particularly questioned and may be, for example, powder, granule (including fine granules), tablet, hard capsule, soft capsule, liquid (e.g., solution, suspension, milky lotion), drink, jelly, pudding, yogurt, candy, chewing gum or the like. These can be produced by a known method. For example, indigestible polysaccharides (e.g., oligosaccharide) are mixed with carriers (e.g., excipient, binder, disintegrant, lubricant, solvent) and powder, granule, tablet, capsule, liquid and the like can be produced by a method known in the field of food preparation or pharmaceutical preparation. In addition, they can also be produced by adding and mixing indigestible polysaccharides (e.g., oligosaccharide) to and with food and drink (e.g., water, soft drink). Commercially available products (e.g., fructo-oligosaccharide "Meiorigo P granule" manufactured by Meiji Food Materia Co., Ltd.) can also be used.

### (2) Indigestible protein or composition containing indigestible protein

In the present specification, indigestible protein refers to a protein that is difficult to digest and absorb in the stomach and small intestine, and reaches the large intestine where it is metabolized by intestinal bacteria such as Clostridium sporogenes and the like.

In the present invention, an example of an indigestible protein is plant protein.

In the present invention, an indigestible protein (e.g., plant protein) can be used as is, or can be used as a composition containing an indigestible protein (e.g., plant protein).

Examples of plant protein or composition containing plant protein include pulverized, refined, and processed products of plants rich in protein (e.g., soybeans, buckwheat, wheat, rice, peas, corn). Examples include soybean protein, buckwheat protein, wheat protein, rice protein, pea protein, corn protein, soy flour, buckwheat flour, wheat flour, rice flour, pea flour, corn flour, soybean processed products (e.g., freeze-dried tofu, miso, miso soup), buckwheat processed products (e.g., buckwheat noodles), wheat processed products, rice processed products (e.g., sake lees), pea processed products (e.g., pea soup), and corn processed products (e.g., corn soup). In the present invention, soybean protein is preferred as the indigestible protein.

In the present invention, the mixing ratio (weight ratio) of (1) indigestible polysaccharides (e.g., fructooligosaccharide) and (2) indigestible protein (e.g., soybean protein) ((1):(2)) is preferably 1:0.1 to 10, more preferably 1:0.5 to 5, particularly preferably 1:1.

### (3) Enteric composition containing amino acid or salt thereof

Examples of the amino acid in the present invention include tryptophan and phenylalanine, and tryptophan and phenylalanine are preferred. These amino acids are preferably in an L form. One or more kinds of amino acids can be used in combination.

As the amino acid in the present invention, it is preferable to contain at least tryptophan, and a combination of tryptophan and phenylalanine is particularly preferred.

The amino acid in the present invention may be in the form of a salt (e.g., sodium salt, hydrochloride). Salts acceptable as medicament or food can be mentioned.

In the present invention, when tryptophan and phenylalanine are used in combination as amino acids, the mixing ratio of tryptophan and phenylalanine (tryptophan:phenylalanine (molar ratio)) is preferably 1:0.01 to 100, more preferably 1:0.1 to 10, further preferably 1:1 to 10, particularly preferably 1:1 to 4.

In the present invention, the enteric composition is a composition that is not disintegrated in the stomach or the upper part of the small intestine where pH is low but disintegrated in the lower part of the small intestine or large intestine where pH is high and releases amino acid or a salt thereof.

The enteric composition in the present invention is, for example, stable at a pH of less than 2, the outermost layer coating peels off at pH 2 or more, after which disintegrates at pH 3.5 or more (more preferably pH 3.5 to pH 9).

The disintegration property can be measured, for example, according to the rules for enteric preparations in the disintegration test method, the Japanese Pharmacopoeia 17th edition.

Examples of the enteric composition in the present invention include granules (including fine granules), granulated substance, tablet, hard capsule and soft capsule.

The enteric composition in the present invention can be produced by a method known in the field of food preparation or pharmaceutical preparation.

For example, a method including mixing, granulating and/or tableting amino acid or a salt thereof together with a carrier (e.g., excipient, binder, disintegrant, lubricant, protector) to give granule or tablet, applying an enteric coating thereon to give enteric granule or enteric tablet; a method including filling granules or tablets containing amino acid or a salt thereof in a hard capsule applied with an enteric coating to give an enteric capsule; and a method including encapsulating a suspension of amino acid or a salt thereof in a carrier (e.g., oil component) in a soft capsule composed of an enteric substrate to give an enteric capsule can be mentioned. In addition, the above-mentioned enteric granules may be encapsulated in a capsule (e.g., cellulose capsule) to give a capsule. Also, a melted oil (protector) may be mixed with an amino acid or a salt thereof, cooled and solidified to give an enteric granulated substance.

Commercially available products can also be used.

Examples of the enteric coating base include cellulose polymers such as hydroxypropylcellulose, hydroxypropylmethylcellulose phthalate, hydroxypropylmethylcellulose acetate succinate, carboxymethylethylcellulose, cellulose acetate phthalate and the like; acrylic acid polymers such as methacrylic acid copolymer L, methacrylic acid copolymer LD, methacrylic acid copolymer S and the like; and naturally occurring substances such as chitosan, shellac and the like. A coating additive (e.g., plasticizer) may also be used during coating.

The carrier, coating base and coating additive are used in amounts conventionally employed in the technical field of preparation formulation.

In the present invention, when (3) an amino acid or a salt thereof is used, the mixing ratio (weight ratio) of (1) an indigestible polysaccharide (e.g., fructooligosaccharide), (2) an indigestible protein (e.g., soybean protein), and (3) an amino acid or a salt thereof (e.g., tryptophan, phenylalanine) ((1):(2):(3)) is preferably 1:0.1 to 10:0.0003 to 1, more preferably 1:0.5 to 5:0.0001 to 0.1, particularly preferably 1:1:0.005 to 0.05.

The ingestion amount of indigestible polysaccharide (e.g., oligosaccharide) or the composition containing an indigestible polysaccharide in the food for improving intestinal environment of the present invention is generally 0.1 - 20 g, preferably 0.1 - 10 g, more preferably 0.1 - 5 g, as indigestible polysaccharide, per day for an adult (body weight 60 kg).

The ingestion amount of indigestible protein (e.g., soybean protein) or the composition containing an indigestible protein in the food for improving the intestinal environment of the present invention is generally 0.1 - 20 g, preferably 0.1 - 10 g, more preferably 0.1 - 5 g, as indigestible protein, per day for an adult (body weight 60 kg).

In the food for improving intestinal environment of the present invention, when amino acid or a salt thereof is used, the ingestion amount of amino acid or a salt thereof in the food for improving intestinal environment of the present invention is generally 0.01 - 10 g, preferably 0.01 - 5 g, more preferably 0.01 - 1 g, as amino acid, per day for an adult (body weight 60 kg).

As another embodiment of the present invention, "a commercial package containing a food containing a combination of (1) an indigestible polysaccharide or a composition containing an indigestible polysaccharide, and (2) an indigestible protein or a composition containing an indigestible protein, and a written matter describing explanation relating to use for improving intestinal environment",
"a commercial package including a food containing a combination of (1) an indigestible polysaccharide or a composition containing an indigestible polysaccharide, (2) an indigestible protein or a composition containing an indigestible protein, and (3) an enteric composition containing an amino acid or a salt thereof, and a written matter describing explanation relating to use for improving intestinal environment" "a food with an indication that it is used for improving intestinal environment, which contains a combination of (1) an indigestible polysaccharide or a composition containing an indigestible polysaccharide, and (2) an indigestible protein or a composition containing an indigestible protein", and "a food with an indication that it is used for improving intestinal environment, which contains a combination of (1) an indigestible polysaccharide or a composition containing an indigestible polysaccharide, (2) an indigestible protein or a composition containing an indigestible protein, and (3) an enteric composition containing an amino acid or a salt thereof" can be mentioned.

The definitions, examples, and ingestion amounts of "an indigestible polysaccharide or a composition containing an indigestible polysaccharide", "an indigestible protein or a composition containing an indigestible protein", and "an enteric composition containing an amino acid or a salt thereof" are the same as those described for the above-mentioned food for improving the intestinal environment.

### [Second Invention]

The food for improving the intestinal environment of the present invention contains at least one selected from the group consisting of an enteric composition containing tryptophan or a salt thereof, an indigestible protein containing tryptophan as a constituent amino acid, and a composition containing an indigestible protein containing tryptophan as a constituent amino acid.

The food for improving the intestinal environment of the present invention is further preferably combined with an enteric composition containing phenylalanine or a salt thereof, and/or an indigestible polysaccharide or a composition containing an indigestible polysaccharide.

In the food for improving the intestinal environment of the present invention, when at least one selected from the group consisting of (I) an enteric composition containing tryptophan or a salt thereof, an indigestible protein containing tryptophan as a constituent amino acid, and a composition containing an indigestible protein containing tryptophan as a constituent amino acid, is used in combination with (II) an enteric composition containing phenylalanine or a salt thereof and/or (III) an indigestible polysaccharide or a composition containing an indigestible polysaccharide, for example, the following embodiments can be mentioned.
(i) A food for improving the intestinal environment, containing a combination of an enteric composition containing tryptophan or a salt thereof and an enteric composition containing phenylalanine or a salt thereof.
(ii) A food for improving the intestinal environment, containing a combination of an indigestible protein (e.g., soybean protein) containing tryptophan as a constituent amino acid and an enteric composition containing phenylalanine or a salt thereof.
(iii) A food for improving the intestinal environment, containing a combination of a composition containing an indigestible protein (e.g., soybean protein) containing tryptophan as a constituent amino acid, and an enteric composition containing phenylalanine or a salt thereof.
(iv) A food for improving the intestinal environment, containing a combination of an enteric composition containing tryptophan or a salt thereof, and an indigestible polysaccharide (e.g., oligosaccharide (preferably, fructooligosaccharide)) or a composition containing an indigestible polysaccharide.
(v) A food for improving the intestinal environment, containing a combination of an indigestible protein (e.g., soybean protein) containing tryptophan as a constituent amino acid, and an indigestible polysaccharide (e.g., oligosaccharide (preferably, fructooligosaccharide)) or a composition containing an indigestible polysaccharide.
(vi) A food for improving the intestinal environment, containing a combination of a composition containing an indigestible protein (e.g., soybean protein) containing tryptophan as a constituent amino acid, and indigestible polysaccharide (e.g., oligosaccharide(preferably, fructooligosaccharide)) or a composition containing an indigestible polysaccharide.
(vii) A food for improving the intestinal environment, containing a combination of an enteric composition containing tryptophan or a salt thereof, an enteric composition containing phenylalanine or a salt thereof, and an indigestible polysaccharide (e.g., oligosaccharide (preferably, fructooligosaccharide)) or a composition containing an indigestible polysaccharide.
(viii) A food for improving the intestinal environment, containing a combination of an indigestible protein (e.g., soybean protein) containing tryptophan as a constituent amino acid, an enteric composition containing phenylalanine or a salt thereof, and an indigestible polysaccharide (e.g., oligosaccharide (preferably, fructooligosaccharide)) or a composition containing an indigestible polysaccharide.
(ix) A food for improving the intestinal environment, containing a combination of a composition containing an indigestible protein (e.g., soybean protein) containing tryptophan as a constituent amino acid, an enteric composition containing phenylalanine or a salt thereof, and an indigestible polysaccharide (e.g., oligosaccharide (preferably, fructooligosaccharide)) or a composition containing an indigestible polysaccharide.

In the food for improving the intestinal environment of the present invention, when (I) at least one selected from the group consisting of an enteric composition containing tryptophan or a salt thereof, an indigestible protein containing tryptophan as a constituent amino acid, and a composition containing an indigestible protein containing tryptophan as a constituent amino acid, is used in combination with (II) an enteric composition containing phenylalanine or a salt thereof and/or (III) an indigestible polysaccharide or a composition containing an indigestible polysaccharide, then (I), (II), and/or (III) may be formulated simultaneously and contained in the same formulation (food), or (I), (II), and/or (III) may be formulated separately and ingested simultaneously or separately with a time difference, via the same or different routes. That is, the food of the present invention includes a food containing (I), (II), and/or (III) in a single formulation, and a food in which (I), (II), and/or (III) are formulated separately and used in combination.

In the present invention, L-forms of tryptophan and phenylalanine are preferred.

In the present invention, tryptophan and phenylalanine may be in the form of salts (e.g., sodium salts, hydrochloride salts). Salts that are acceptable as medicaments or foods can be mentioned.

In the present invention, when (I) at least one selected from the group consisting of an enteric composition containing tryptophan or a salt thereof, an indigestible protein containing tryptophan as a constituent amino acid, and a composition containing an indigestible protein containing tryptophan as a constituent amino acid, is used in combination with (II) an enteric composition containing phenylalanine or a salt thereof, the mixing ratio of (I) and (II) is preferably 1:0.01 to 100, more preferably 1:0.1 to 10, further preferably 1:1 to 10, particularly preferably 1:1 to 4, as a molar ratio of tryptophan in (I) to phenylalanine in (II) (tryptophan:phenylalanine).

In the present invention, when (I) at least one selected from the group consisting of an enteric composition containing tryptophan or a salt thereof, an indigestible protein containing tryptophan as a constituent amino acid, and a composition containing an indigestible protein containing tryptophan as a constituent amino acid, is used in combination with (III) an indigestible polysaccharide or a composition containing an indigestible polysaccharide, the mixing ratio of (I) and (III) is preferably 1:1 to 3000, more preferably 1:10 to 2000, further preferably 1:20 to 1000, particularly preferably 1:30 to 500, as a weight ratio of tryptophan in (I) to indigestible polysaccharide (e.g., oligosaccharide (preferably fructooligosaccharide)) in (III) (tryptophan:indigestible polysaccharide).

In the present invention, when (I) at least one selected from the group consisting of an enteric composition containing tryptophan or a salt thereof, an indigestible protein containing tryptophan as a constituent amino acid, and a composition containing an indigestible protein containing tryptophan as a constituent amino acid, is used in combination with (II) an enteric composition containing phenylalanine or a salt thereof, and (III) an indigestible polysaccharide or a composition containing an indigestible polysaccharide, the mixing ratio of (I), (II), and (III) is preferably 1:0.01 to 100:1 to 3000, more preferably 1:0.05 to 10:10 to 2000, further preferably 1:0.1 to 10:20 to 1000, particularly preferably 1:0.5 to 4:30 to 500, as a weight ratio of tryptophan in (I), phenylalanine in (II), and indigestible polysaccharide (e.g., oligosaccharide (preferably fructooligosaccharide)) in (III) (tryptophan:phenylalanine:indigestible polysaccharide).

In the present invention, the definition, examples, and production methods of "enteric composition" in an enteric composition containing tryptophan and an enteric composition containing phenylalanine, the definition, degree of polymerization, and examples of indigestible polysaccharides (oligosaccharide), and examples of a composition containing an indigestible polysaccharide, are the same as the definition, examples, and production methods of "enteric composition", the definition, degree of polymerization, and examples of indigestible polysaccharide (oligosaccharide), and the examples of a composition containing an indigestible polysaccharide described in the above-mentioned first invention.

In the present invention, examples of the "indigestible protein containing tryptophan as a constituent amino acid" include plant proteins containing tryptophan as a constituent amino acid. In the present invention, examples of the "composition containing indigestible proteins containing tryptophan as a constituent amino acid" include compositions containing plant proteins containing tryptophan as a constituent amino acid.

Examples of the plant proteins containing tryptophan as a constituent amino acid, or compositions containing plant proteins containing tryptophan as a constituent amino acid, include pulverized, refined, and processed products of plants rich in protein (e.g., soybeans, buckwheat, wheat, rice, peas, corn). Examples include soybean protein, buckwheat protein, wheat protein, rice protein, pea protein, corn protein, soy flour, buckwheat flour, wheat flour, rice flour, pea flour, corn flour, soybean processed products (e.g., freeze-dried tofu, miso, miso soup), buckwheat processed products (e.g., buckwheat noodles), wheat processed products, rice processed products (e.g., sake lees), pea processed products (e.g., pea soup), and corn processed products (e.g., corn soup). In the present invention, soybean protein is preferred as the indigestible protein.

In the food for improving the intestinal environment of the present invention, the ingestion amount of "at least one selected from the group consisting of an enteric composition containing tryptophan or a salt thereof, an indigestible protein containing tryptophan as a constituent amino acid, and a composition containing an indigestible protein containing tryptophan as a constituent amino acid" is generally 0.01 to 5 g, preferably 0.01 to 1 g, more preferably 0.01 to 0.5 g, as tryptophan, per day for an adult (body weight 60 kg).

In the food for improving the intestinal environment of the present invention, when "an enteric composition containing phenylalanine or a salt thereof" is used, the ingestion amount of "an enteric composition containing phenylalanine or a salt thereof" in the food for improving the intestinal environment of the present invention is generally 0.01 to 5 g, preferably 0.01 to 1 g, more preferably 0.01 to 0.5 g, as phenylalanine, per day for an adult (body weight 60 kg).

In the food for improving the intestinal environment of the present invention, when "an indigestible polysaccharide or a composition containing an indigestible polysaccharide" is used, the ingestion amount of "an indigestible polysaccharide or a composition containing an indigestible polysaccharide" in the food for improving intestinal environment of the present invention is generally 0.1 - 20 g, preferably 0.5 - 10 g, more preferably 1 - 5 g, as indigestible polysaccharide, per day for an adult (body weight 60 kg).

As other embodiments of the present invention, "a commercial package including a food containing at least one selected from the group consisting of an enteric composition containing tryptophan or a salt thereof, an indigestible protein containing tryptophan as a constituent amino acid, and a composition containing an indigestible protein containing tryptophan as a constituent amino acid, and a written matter describing explanation relating to use for improving intestinal environment",
"a commercial package including a food containing a combination of (I) at least one selected from the group consisting of an enteric composition containing tryptophan or a salt thereof, an indigestible protein containing tryptophan as a constituent amino acid, and a composition containing an indigestible protein containing tryptophan as a constituent amino acid, and (II) an enteric composition containing phenylalanine or a salt thereof and/or (III) an indigestible polysaccharide or a composition containing an indigestible polysaccharide, and a written matter describing explanation relating to use for improving intestinal environment",
"a food with an indication that it is used for improving the intestinal environment, containing at least one selected from the group consisting of an enteric composition containing tryptophan or a salt thereof, an indigestible protein containing tryptophan as a constituent amino acid, and a composition containing an indigestible protein containing tryptophan as a constituent amino acid", and
"a food with an indication that it is used for improving the intestinal environment, containing a combination of (I) at least one selected from the group consisting of an enteric composition containing tryptophan or a salt thereof, an indigestible protein containing tryptophan as a constituent amino acid, and a composition containing an indigestible protein containing tryptophan as a constituent amino acid, and (II) an enteric composition containing phenylalanine or a salt thereof and/or (III) an indigestible polysaccharide or a composition containing an indigestible polysaccharide" can be mentioned.

The definitions, examples, and ingestion amounts of "an enteric composition containing tryptophan or a salt thereof", "an indigestible protein containing tryptophan as a constituent amino acid", "a composition containing an indigestible protein containing tryptophan as a constituent amino acid", "an enteric composition containing phenylalanine or a salt thereof", and "an indigestible polysaccharide or a composition containing an indigestible polysaccharide" are the same as those described for the above-mentioned food for improving the intestinal environment.

The food (first invention and second invention) of the present invention can be safely given to human and animals other than human (e.g., mammals and birds such as domestic animals, poultry, experiment animals and the like). The ingestion form for animals other than human may be addition to a feed.

In the present specification, "intestinal environment" means "an intestinal environment in which IPA is easily produced".

In the present specification, "improvement of the intestinal environment" means an increase in IPA production by intestinal bacteria in the intestine; specifically, an increase in IPA production by intestinal bacteria in the intestine when the food of the present invention is consumed compared to when the food of the present invention is not consumed (resulting in increased blood IPA concentration of the host).

In the present specification, "for improving the intestinal environment" means being used to increase IPA production by intestinal bacteria in the intestine by adjusting the intestinal environment to a state in which IPA is easily produced.

In the present specification, "improvement of intestinal permeability" means bringing increased intestinal permeability closer to normal. Intestinal permeability can be confirmed according to the method described in the below-mentioned Experimental Example 5.

In the present specification, "food" is a concept widely encompassing those that can be taken orally (excluding pharmaceuticals), and includes not only so-called "foods" but also beverages, health supplements, health functional foods (e.g., Foods for Specified Health Uses, Foods with Function Claims), supplements, and the like.

Examples of foods in the present invention (first invention and second invention) include soup, miso soup, meat substitute, protein bar, and the like.

### [Example]

The present invention is described more specifically below with reference to Experimental Examples, but the present invention is not limited to these Experimental Examples.

Abbreviations mean the following.
Trp: L-tryptophan
Phe: L-phenylalanine
Gly: glycine
Ile: L-isoleucine
Val: L-valine
Ala: L-alanine
Arg: L-arginine
Asn: L-asparagine
Asp: L-aspartic acid
Cys: L-cysteine
Gln: L-glutamine
Glu: L-glutamic acid
His: L-histidine
Leu: L-leucine
Lys: L-lysine
Met: L-methionine
Pro: L-proline
Ser: L-serine
Thr: L-threonine
Tyr: L-tyrosine
IMOS: isomaltooligosaccharide
GOS: galactooligosaccharide
FOS: fructooligosaccharide
PBS: phosphate buffered saline
C. sporogenes: Clostridium sporogenes
RS: resistant starch
HFD: high-fat diet
SPI: soybean protein isolate
LFD: low-fat diet

### [Experimental Example 1-1] Culture test of C. sporogenes in the presence of various amino acids (Gly, Ile, Phe, Trp, Val)

Amino acids that promote the growth of an IPA-producing bacterium C. sporogenes were narrowed down using a genome-scale metabolic model (GEM) simulation, and then evaluated in a pure culture system. The GEM simulation was performed using COBRA toolbox of MATLAB (registered trademark), and amino acids that affected the growth of C. sporogenes when 20 types of amino acids were removed one by one were selected. Then, the influence of amino acids selected by GEM analysis on the growth of C. sporogenes was investigated in a pure culture system. As C. sporogenes, an in-house strain from Ajinomoto Co., Inc. was used, and as the culture medium, 1/5 diluted enhanced Clostridium medium (Difico, #62620609) was used. Each amino acid was added to the medium to a final concentration of 4 mM, and the growth of C. sporogenes was evaluated using OD600 as an indicator. The case without addition of amino acid (culture medium only) was used as a control.

The results are shown in Fig. 1-1, and Fig. 1-2.

From the results of Fig. 1-1, the GEM simulation showed that the growth of Gly, Ile, Phe, Trp, and Val decreased by 10% or more compared to before the removal of the amino acids (Fig. 1-1). The influence of these amino acids on the growth of C. sporogenes was evaluated in a pure culture system, and it was shown that the addition of Phe promoted the growth of C. sporogenes (Fig. 1-2, in the Figure, *** indicates p<0.001, significance test with control by Dunnett, n=6, means ± SD). Therefore, it was shown that phenylalanine is essential for promoting the growth of C. sporogenes.

### [Experimental Example 1-2] Effect of combined use of tryptophan and phenylalanine on IPA production

### (Test method)

To evaluate the influence of Trp and a combination of Trp and Phe on IPA production, pure culture experiments using C. sporogenes were performed. As C. sporogenes, in-house strain of Ajinomoto Co., Inc. was used. First, 800 µL of C. sporogenes glycerol stock was added to 40 mL of reinforced Clostridium medium and cultured under anaerobic conditions at 37°C for 18 hr. After culturing, centrifugation was performed at 9,100 × g for 5 min at 4°C, and the precipitate was collected under anaerobic conditions, washed twice with PBS, and finally suspended in 5 mL of PBS after anaerobic treatment to obtain a concentrated bacterial solution. The influence of the concentrated bacterial solution on IPA production was investigated. The Trp concentrations used to evaluate the influence of Trp on IPA production are shown in Table 1. In addition, the Trp or Phe concentrations used to evaluate the influence of Trp and Phe combination on IPA production are shown in Table 2. To 500 µL of the concentrated bacterial suspension, 10 mM Trp or 10 mM Phe was added in the amounts shown in Table 1 or Table 2. The total volume was adjusted to 1 mL using PBS, and the suspension was cultured at 37°C under anaerobic conditions for 24 hr. Furthermore, similar to Experimental Example 1, the influence of Phe/Trp molar ratios between 1 and 10 on IPA production in C. sporogenes was evaluated using GEM analysis.

### (Method for analyzing IPA concentration)

The IPA concentration in the culture supernatant was analyzed using an LC-MS/MS mass spectrometry system (mass spectrometer: SCIEX API3200, pump: Shimadzu Corporation LC-20AD, autosampler: Shimadzu Corporation SIL-20AC, column oven: Shimadzu Corporation CTO-20AC, system controller: Shimadzu Corporation CBM-20A, degasser: Shimadzu Corporation DGU-20A, nitrogen generator: Airtech AT5NP-25CSL). Separation was performed by injecting 5 µL of the sample and then using an L-Column 2 (2.1 × 150 mm, 3 µm, Chemicals Evaluation and Research Institute) column, with 0.2% acetic acid (A) and 0.2% acetic acid/acetonitrile solution (B) as the mobile phases, at a constant flow rate (0.2 mL/min) under conditions where the mobile phase B concentration was 2% (1 min), 10% (1.1 min), 25% (3 min), 50% (4 min), 70% (7 min), 95% (9.5-12.5 min), and 2% (12.6-19 min). Mass measurements were performed under the following conditions: CUR: 30 psi, IS: 5500 V, TEM: 700°C, GAS1: 70 psi, GAS2: 70 psi, CAD: 4, CXP: 4 V, ionization method: ESI(+). The peak area ratios of standard solutions of IPA (Tokyo Chemical Industries, P/N: I0032) with known concentrations (six concentrations ranging from 0.002 to 0.5 µM) were calculated, and the concentrations were determined based on the standard curves produced from these ratios.

**[Table 1]**

| Concentration of Trp used for evaluating influence of Trp on IPA production | | | |
|---|---|---|---|
| Group | necessary amount (µL) | | Trp concentration (mM) |
| | 10 mM Trp | PBS | |
| Control | 0 | 500 | 0.00 |
| final concentration 500 µM Trp | 50 | 450 | 0.50 |
| final concentration 100 µM Trp | 10 | 490 | 0.10 |
| final concentration 50 µM Trp | 5 | 495 | 0.05 |
| final concentration 10 µM Trp | 1 | 499 | 0.01 |

**[Table 2]**

| Concentration of Trp or Phe used for evaluating influence of Trp and Phe in combination on IPA production | | | | | | | |
|---|---|---|---|---|---|---|---|
| Group | necessary amount (µL) | | | concentration (mM) | | molar ratio | |
| | 10 mM Trp | 10 mM Phe | PBS | Trp | Phe | Trp | Phe |
| Control | 0 | 0 | 500 | 0.0 | 0.0 | 0 | 0 |
| Phe alone | 0 | 30 | 470 | 0.0 | 0.3 | 0 | 1 |
| Trp alone | 30 | 0 | 470 | 0.3 | 0.0 | 1 | 0 |
| Trp:Phe=1:1 | 30 | 30 | 440 | 0.3 | 0.3 | 1 | 1 |
| Trp: Phe=1:2 | 30 | 60 | 410 | 0.3 | 0.6 | 1 | 2 |
| Trp: Phe=1:3 | 30 | 90 | 380 | 0.3 | 0.9 | 1 | 3 |
| Trp:Phe=1:4 | 30 | 120 | 350 | 0.3 | 1.2 | 1 | 4 |

The results are shown in Fig. 1-3. In the Figure, 1:1, 1:2, 1:3, and 1:4 represent Trp:Phe. From the results of Fig. 1-3, no effect on IPA production was observed when Trp was absent from the culture medium and Phe alone was present. On the other hand, when the Trp concentration was fixed and the molar ratio of Phe to Trp was increased, an increase in IPA in the culture supernatant was observed in a Phe molar ratio-dependent manner. Furthermore, the results of the GEM analysis are shown in Fig. 1-4. From the results of Fig. 1-4, it was found that IPA production by C. sporogenes is maximized at a Phe/Trp molar ratio of 4.

### [Experimental Example 2-1] Evaluation of sugar sources that enhance IPA production (in vivo test using mice)

### (Test method)

C57BL/6J mice (Charles River Co., Ltd., Japan) aged 10-20 weeks were used and raised with one mouse per cage. The test sugar source materials, fructooligosaccharide (FUJIFILM Wako Pure Chemical Corporation, #064-02385), galactooligosaccharide (FUJIFILM Wako Pure Chemical Corporation, #076-05945), and isomaltooligosaccharide (FUJIFILM Wako Pure Chemical Corporation, #090-03485), were mixed with powdered feed AIN93G (Research Diet) in the mixing ratios shown in Table 3. About 10% water was added and kneaded, then the mixture was formed into about 5 g balls and dried at room temperature for two days. Mice were fasted from 4 p.m. the previous day, blood was collected at 4 p.m. on the day of the experiment, and then fed until 9 a.m. the following day. After collecting the feed, blood was collected. The blood was converted into plasma, and the IPA concentration was measured. The change in IPA (ΔIPA) was compared.

**[Table 3]**

| Mixing ratio of sugar source materials in feed (w/w) | | | | |
|---|---|---|---|---|
| group name | AIN93G | IMOS | GOS | FOS |
| IMOS group | 97.5% | 2.5% | - | - |
| GOS group | 97.5% | - | 2.5% | - |
| FOS group | 97.5% | - | - | 2.5% |
| AIN93G group | 100.0% | - | - | - |

The results are shown in Fig. 2-1. From the results of Fig. 2-1, it was shown that FOS intake resulted in a significant increase in blood IPA levels.

### [Experimental Example 2-2] Evaluation of sugar sources that enhance IPA production (in vitro test using human fecal suspension)

### (Test method)

Oligosaccharides (fructooligosaccharide (FUJIFILM Wako Pure Chemical Corporation, #064-02385), galactooligosaccharide (FUJIFILM Wako Pure Chemical Corporation, #076-05945), isomaltooligosaccharide (FUJIFILM Wako Pure Chemical Corporation, #090-03485)) were prepared as PBS solutions according to the formulations shown in Table 4. Each solution was filtered and sterilized, and 5 mL of each solution was dispensed into sterile glass vials and subjected to anaerobic conditions in an anaerobic chamber. Feces were collected from six healthy individuals aged 20-40 (3 men, 3 women). In an anaerobic chamber, about 1 g of each feces was suspended in 10 mL of anaerobic PBS to prepare a fecal suspension. 0.1 mL of this fecal suspension was added to each vial, sealed with a butyl rubber stopper, and incubated anaerobically for 3 days in a 37°C incubator, with inversion and mixing once daily. After incubation, a portion of the culture medium was centrifuged (8,000 × g, 5 min), and the IPA concentration in the culture supernatant was measured. The data were standardized based on the fecal results of each subject and are shown as the mean±standard error of the z-score (n=6).

**[Table 4]**

| Mixing ratio of sugar source materials in culture medium (w/w) | | | | |
|---|---|---|---|---|
| group name | Trp | IMOS | GOS | FOS |
| IMOS group | - | 0.2% | - | - |
| GOS group | - | - | 0.2% | - |
| FOS group | - | - | - | 0.2% |
| PBS group | - | - | - | - |
| IMOS+Trp group | 0.01% | 0.2% | - | - |
| GOS+Trp group | 0.01% | - | 0.2% | - |
| FOS+Trp group | 0.01% | - | - | 0.2% |
| PBS+Trp group | 0.01% | - | - | - |

The results are shown in Fig. 2-2. From the results of Fig. 2-2, the IPA concentration in the culture medium hardly increased with the addition of oligosaccharides alone, but the IPA concentration in the culture medium could be increased by using oligosaccharides and Trp in combination. The effect thereof was most remarkable when FOS and Trp were used in combination.

### [Experimental Example 3] Evaluation of protein that enhances IPA production (in vitro test using human fecal suspension)

### (Test method)

C57BL/6J mice (Charles River Co., Ltd., Japan) aged 10-20 weeks were used and raised with one mouse per cage. The test protein materials, soybean protein (Fuji Oil Co., Ltd., Prolina (isolated soybean protein)), whey (Fonterra, WPC), and casein (Fonterra, sodium caseinate), were mixed with powdered feed AIN93G (Research Diet) in the mixing ratios shown in Table 5. About 10% water was added and kneaded, then the mixture was formed into about 5 g balls and dried at room temperature for two days. Mice were fasted from 4 p.m. the previous day, blood was collected at 4 p.m. on the day of the experiment, and then fed until 9 a.m. the following day. After collecting the feed, blood was collected. The blood was converted into plasma, and the IPA concentration was measured. The change in IPA (ΔIPA) was compared.

**[Table 5]**

| Mixing ratio of protein materials in feed (w/w) | | | | |
|---|---|---|---|---|
| group name | AIN93G | soybean protein | whey | casein |
| soybean protein group | 80% | 20% | - | - |
| whey group | 80% | - | 20% | - |
| casein group | 80% | - | - | 20% |
| AIN93G group | 100% | - | - | - |

The results are shown in Fig. 3. From the results of Fig. 3, it was shown that soybean protein intake resulted in a significant increase in blood IPA levels.

### [Experimental Example 4] Mixing ratio of fructooligosaccharide and soybean protein

### (Test method)

C57BL/6J mice (Charles River Co., Ltd., Japan) aged 10-20 weeks were used and raised with one mouse per cage. The test sugar source material, fructooligosaccharide (FUJIFILM Wako Pure Chemical Corporation, #064-02385), and the test protein material, soybean protein (Fuji Oil Co., Ltd., Prolina (isolated soybean protein)) were mixed with powdered feed AIN93G (Research Diet) in the mixing ratios shown in Table 6. About 10% water was added and kneaded, then the mixture was formed into about 5 g balls and dried at room temperature for two days. Mice were fasted from 4 p.m. the previous day, blood was collected at 4 p.m. on the day of the experiment, and then fed until 9 a.m. the following day. After collecting the feed, blood was collected. The blood was converted into plasma, and the IPA concentration was measured. The change in IPA (ΔIPA) was compared.

**[Table 6]**

| Mixing ratio of soybean protein and FOS in feed (w/w) | | | |
|---|---|---|---|
| group name | AIN93G | soybean protein | FOS |
| soybean protein group | 90% | 10% | - |
| soybean protein :FOS=3:1 group | 90% | 7.5% | 2.5% |
| soybean protein :FOS=1:1 group | 90% | 5% | 5% |
| soybean protein :FOS=1:3 group | 90% | 2.5% | 7.5% |
| FOS group | 90% | - | 10.0% |
| AIN93G group | 100% | - | - |

The results are shown in Fig. 4. From the results of Fig. 4, combining soybean protein and FOS remarkably increased blood IPA concentration compared to ingesting either soybean protein or FOS alone. This indicates a synergistic effect of combining soybean protein and FOS. In particular, a soybean protein:FOS ratio of 1:1 was shown to be optimal for intestinal IPA production.

### [Experimental Example 5] Long-term test

### (Test method)

C57BL/6J mice (Charles River Co., Ltd., Japan) aged 15 weeks were used and raised with one mouse per cage. The test sugar source material, fructooligosaccharide (FUJIFILM Wako Pure Chemical Corporation, #064-02385), and the test protein material, soybean protein (Fuji Oil Co., Ltd., Prolina (isolated soybean protein)), and resistant starch (J-Oil Mills, Amylofiber SH) as a comparison material were mixed with powdered high-fat feed (Research Diet, 60 kcal Fat, #D12492) in the mixing ratios shown in Table 7. About 10% water was added and kneaded, then the mixture was formed into about 5 g balls and dried at room temperature for two days. In addition, 15% cellulose was added to the high-fat control feed to match the fat content of the other feeds, about 10% water was added and kneaded, then the mixture was formed into about 5 g balls and dried at room temperature for two days. Furthermore, the normal control diet was prepared by adding about 10% water to powdered low-fat feed (Research Diet, 10 kcal Fat, #D12450B), kneading and forming the mixture into about 5 g balls, and drying them at room temperature for two days. These diets were freely ingested by the mice, and blood samples were collected and intestinal permeability tests were performed after 4 weeks. The blood was converted into plasma, and the IPA concentration was measured. For the intestinal permeability test, to the mice, following fasting for 24 hr, were orally administered 0.5 mL of FITC fluorescently labeled 4,000 Da dextran (Sigma-Aldrich, #FD4) at a concentration of 22 mg/mL, and blood samples were collected 4 hr later. Blood was centrifuged at 12,000 × g, and the concentration of FITC-labeled dextran in the plasma was measured using a fluorescence plate reader (Molecular Devices, SpectraMax) under the conditions of excitation wavelength 485 nm and emission wavelength 530 nm. The concentration was determined based on a standard curve produced from the fluorescence intensity of FITC-labeled dextran with known concentration.

**[Table 7]**

| Mixing ratio of soybean protein and FOS in feed (w/w) | | | | | | |
|---|---|---|---|---|---|---|
| group name | high-fat feed | low-fat feed | cellulose | soybean protein | FOS | RS |
| high-fat control group | 85% | - | 15% | - | - | - |
| soybean protein: FOS=1:1 group | 85% | - | - | 7.5% | 7.5% | - |
| RS group | 85% | - | - | - | - | 15% |
| normal control group | - | 100% | - | - | - | - |

The results are shown in Fig. 5. In the Figure, HFD represents the high-fat control group, SPI+FOS represents the soybean protein:FOS = 1:1 group, RS represents the RS group, and LFD represents the normal control group.

From the results of Fig. 5, the combination of soybean protein and FOS showed an effect of increasing blood IPA and improving intestinal permeability that was equivalent to or greater than that of resident starch (positive control).

### [Experimental Example 6] Examination of Trp colonic delivery formulation

100 mg of Trp and 150 mg of excipients (mixture of sorbitol, crystalline cellulose, hydroxypropyl cellulose, fine silicon dioxide, hydrogenated rapeseed oil, and calcium stearate) were mixed to produce tablets. Furthermore, these tablets were coated using a spray-type rotary tablet coating device: 1st layer: hydroxypropyl cellulose (CELNY (registered trademark), manufactured by Nippon Soda Co., Ltd.), 2nd layer: chitosan (Chitocoat (registered trademark), manufactured by Freund Industrial Co., Ltd.), and 3rd layer: shellac (AQshellax (registered trademark), manufactured by Freund Industrial Co., Ltd.). The Trp colonic delivery formulation produced in this manner was subjected to a disintegration test in accordance with the Japanese Pharmacopoeia. Specifically, the tablets were immersed in the 1st fluid for disintegration test for 2 hr (without an auxiliary disc), then in the 2nd fluid for disintegration test for 2 hr (with an auxiliary disc), and then in the large intestine simulation solution (with an auxiliary disc), and the time up to the disintegration of the tablets was examined. The large intestine simulation solution used was an acetate buffer solution with a pH of 3.5 (Michaelis buffer). A DT110 (manufactured by Freund Industrial Co., Ltd.) was used as the disintegration test apparatus. The apparatus was set to 37°C and 30 cycles/min.

The results are shown in Table 8.

**[Table 8]**

| | 1st fluid for disintegration test (pH 1.2), 2 hr, no auxiliary disc | 2nd fluid for disintegration test (pH 6.8), 2 hr, with auxiliary disc | large intestine simulation solution (pH 3.5), with auxiliary disc |
|---|---|---|---|
| disintegration results | 6 tablets no disintegration | 6 tablets no disintegration | 6 tablets all disintegrated in 1.5 hr |

From the results in Table 8, it was shown that a Trp large intestine delivery formulation can be produced, and that the formulation does not disintegrate in the stomach and small intestine but disintegrates after reaching the large intestine, and Trp can be delivered to the large intestine.

### [Industrial Applicability]

According to the present invention, by forming a food (including beverages and formulations) containing a combination of indigestible polysaccharides (preferably fructooligosaccharide) and indigestible protein (preferably soybean protein), a food (including beverages and formulations) that improves the intestinal environment to a state where IPA is easily produced can be provided.

According to the present invention, by forming a food (including beverages and formulations) containing an enteric composition of tryptophan (particularly a food containing an enteric composition combining tryptophan and phenylalanine, or a food containing a combination of an enteric composition of tryptophan and indigestible polysaccharides (preferably fructooligosaccharide)) (including beverages and formulations), a food (including beverages and formulations) that improves the intestinal environment to a state where IPA is easily produced can be provided.

This application is based on patent application No. 2023-173408 filed in Japan, the contents of which are encompassed in full herein.

## Claims

1. A food for improving the intestinal environment, comprising a combination of (1) an indigestible polysaccharide or a composition containing an indigestible polysaccharide and (2) an indigestible protein or a composition containing an indigestible protein.

2. The food according to claim 1, further comprising (3) an enteric composition containing an amino acid or a salt thereof in combination.

3. The food according to claim 1, wherein the indigestible polysaccharide or the composition containing an indigestible polysaccharide is oligosaccharide or a composition containing oligosaccharide.

4. The food according to claim 1, wherein the indigestible protein or the composition containing an indigestible protein is a plant protein or a composition containing a plant protein.

5. The food according to claim 1, wherein the indigestible protein or the composition containing an indigestible protein is selected from the group consisting of soybean protein, sake lees, freeze-dried tofu, buckwheat, wheat, rice, peas, and corn.

6. The food according to claim 2, wherein the amino acid is tryptophan, or a combination of tryptophan and phenylalanine.

7. The food according to any one of claims 1 to 6, for increasing the production of indole-3-propionic acid by intestinal bacteria in the intestine.

8. A food for improving the intestinal environment, comprising at least one selected from the group consisting of an enteric composition containing tryptophan or a salt thereof, an indigestible protein containing tryptophan as a constituent amino acid, and a composition containing an indigestible protein containing tryptophan as a constituent amino acid.

9. The food according to claim 8, further comprising an enteric composition containing phenylalanine or a salt thereof in combination.

10. The food according to claim 8, wherein the indigestible protein containing tryptophan as a constituent amino acid, or the composition containing an indigestible protein containing tryptophan as a constituent amino acid is selected from the group consisting of soybean protein, sake lees, freeze-dried tofu, buckwheat, wheat, rice, peas, and corn.

11. The food according to claim 8, further comprising an indigestible polysaccharide or a composition containing an indigestible polysaccharide in combination.

12. The food according to any one of claims 8 to 11, for increasing the production of indole-3-propionic acid by intestinal bacteria in the intestine.

13. A method for improving the intestinal environment in a subject, comprising administering an effective amount of a food comprising a combination of (1) an indigestible polysaccharide or a composition containing an indigestible polysaccharides, and (2) an indigestible protein or a composition containing an indigestible protein to the subject.

14. The method according to claim 13, wherein the food is further combined with (3) an enteric composition containing an amino acid or a salt thereof.

15. The food according to claim 13, wherein the indigestible polysaccharide or the composition containing an indigestible polysaccharide is oligosaccharide or a composition containing oligosaccharide.

16. The method according to claim 13, wherein the indigestible protein or the composition containing an indigestible protein is a plant protein or a composition containing a plant protein.

17. The method according to claim 13, wherein the indigestible protein or the composition containing an indigestible protein is selected from the group consisting of soybean protein, sake lees, freeze-dried tofu, buckwheat, wheat, rice, peas, and corn.

18. The method according to claim 14, wherein the amino acid is tryptophan, or a combination of tryptophan and phenylalanine.

19. The method according to any one of claims 13 to 18, for increasing the production of indole-3-propionic acid by intestinal bacteria in the intestine.

20. A method for improving the intestinal environment in a subject, comprising administering an effective amount of a food containing at least one selected from the group consisting of an enteric composition containing tryptophan or a salt thereof, an indigestible protein containing tryptophan as a constituent amino acid, and a composition containing an indigestible protein containing tryptophan as a constituent amino acid.

21. The method according to claim 20, wherein the food is further combined with an enteric composition containing phenylalanine or a salt thereof.

22. The method according to claim 20, wherein the indigestible protein containing tryptophan as a constituent amino acid, or the composition containing an indigestible protein containing tryptophan as a constituent amino acid, is selected from the group consisting of soybean protein, sake lees, freeze-dried tofu, buckwheat, wheat, rice, peas, and corn.

23. The method according to claim 20, wherein the food is further combined with an indigestible polysaccharide or a composition containing an indigestible polysaccharide.

24. The method according to any one of claims 20 to 23, for increasing the production of indole-3-propionic acid by intestinal bacteria in the intestine.

25. Use of a food comprising a combination of (1) an indigestible polysaccharide or a composition containing an indigestible polysaccharide, and (2) an indigestible protein or a composition containing an indigestible protein, for improving the intestinal environment.

26. The use according to claim 25, wherein the food is further combined with (3) an enteric composition containing an amino acid or a salt thereof.

27. The food according to claim 25, wherein the indigestible polysaccharide or the composition containing an indigestible polysaccharide is oligosaccharide or a composition containing oligosaccharide.

28. The use according to claim 25, wherein the indigestible protein or the composition containing an indigestible protein is a plant protein or a composition containing a plant protein.

29. The use according to claim 25, wherein the indigestible protein or the composition containing an indigestible protein is selected from the group consisting of soybean protein, sake lees, freeze-dried tofu, buckwheat, wheat, rice, peas, and corn.

30. The use according to claim 26, wherein the amino acid is tryptophan, or a combination of tryptophan and phenylalanine.

31. The use according to any one of claims 25 to 30, for increasing the production of indole-3-propionic acid by intestinal bacteria in the intestine.

32. Use of a food comprising at least one selected from the group consisting of an enteric composition containing tryptophan or a salt thereof, an indigestible protein containing tryptophan as a constituent amino acid, and a composition containing an indigestible protein containing tryptophan as a constituent amino acid, for improving the intestinal environment.

33. The use according to claim 32, wherein the food is further combined with an enteric composition containing phenylalanine or a salt thereof.

34. The use according to claim 32, wherein the indigestible protein containing tryptophan as a constituent amino acid, or a composition containing an indigestible protein containing tryptophan as a constituent amino acid, is selected from the group consisting of soybean protein, sake lees, freeze-dried tofu, buckwheat, wheat, rice, peas, and corn.

35. The use according to claim 32, wherein the food is further combined with an indigestible polysaccharide or a composition containing an indigestible polysaccharide.

36. The use according to any one of claims 32 to 35, for increasing the production of indole-3-propionic acid by intestinal bacteria in the intestine.
